# EUROPEAN PATENT SPECIFICATION

(11) **EP 3 308 834 B1**
(45) Date of publication and mention of the grant of the patent: **09.01.2019**
(21) Application number: 16193327.0
(22) Date of filing: 11.10.2016
(51) Int. Cl.: A61N 5/10, G01R 33/3873

(54) **PARTICLE THERAPY APPARATUS COMPRISING AN MRI**
PARTIKELTHERAPIEVORRICHTUNG MIT EINEM MRT
APPAREIL DE THÉRAPIE PAR PARTICULES COMPRENANT UN IRM

(43) Date of publication of application: 18.04.2018
(73) Proprietor: Ion Beam Applications, 1348 Louvain-la-Neuve (BE)
(72) Inventor: PRIEELS, Damien, 1348 Louvain-la-Neuve (BE); BRUSASCO, Caterina, 1348 Louvain-la-Neuve (BE)
(74) Representative: De Groote, Christophe

(56) References cited:
- EP-A1- 2 196 241
- GB-A- 2 507 792
- KR-A- 20150 049 317
- KR-A- 20150 060 387
- US-A1- 2010 239 066

## Description

### Field of the invention

The invention relates to an apparatus for irradiating a target with a charged particle beam for therapy purposes and comprising a magnetic resonance imaging (MRI) system for imaging the target.

### Background of the invention

Conventional particle therapy apparatus allows irradiating a target volume of a patient with a beam of energetic charged particles such as protons. While travelling through the patient, the charged particle beams loses energy and the majority of the energy of the particle beam is deposited at the end of the beam path that is called the Bragg peak. This allows an accurate delivery of high dose of energy in a target volume while minimizing the dose of energy delivered to surrounding tissues. The high dose of energy delivered induces damages to the DNA of cells, killing them or stopping their reproduction. Charged particle therapy is therefore used in the field of cancer treatment, as cancerous cells are particularly vulnerable to attacks on DNA.

One of the known drawback of the charged particle therapy is that even small movements of the patient, such as breathing movements, can lead to significant deviations of the delivered dose in the course of the irradiation of the target with the beam. Therefore, it is desirable to use real-time imaging to track the target and to adapt the beam to the motion of organs and to the target. One of the real-time imaging systems that can be used is a magnetic resonance imaging (MRI) system.

Such particle therapy apparatus comprising a MRI system is known from prior art and for example from WO2015/197475. This document discloses a particle therapy apparatus including a conventional rotatable gantry that comprises a plurality of bending magnets to bend and direct the particle beam towards the target (towards the isocentre) in a direction perpendicular to the axis of rotation of the gantry. The MRI system is arranged in such a way that the direction of its main magnetic field (Bo) is the same as the direction of the axis of rotation of the gantry, which is generally chosen to be a horizontal direction for the comfort of the patient who can then be placed horizontally in the particle therapy apparatus.

Also known from document EP2196241 is a particle therapy apparatus wherein the main magnetic field of the MRI system is in the vertical direction and is combined with a fixed beamline, wherein the beam is entering the MRI system through an opening at the top of the MRI system. This arrangement reduces the influence of the magnetic field of the MRI magnets on the path of charged particles. The subject support is adapted for rotational motion during operation of the therapeutic apparatus.

US2010/0239066 describes a particles therapy apparatus that generates a treatment beam and a magnetic field disposed parallel to the treatment beam, wherein a target that is disposed along the treatment beam. The treatment beam can be a proton beam and the magnetic field can be from a MRI system. The magnetic field may operate in coordination with operation of the treatment beam and will narrow said beam. The apparatus generating both said treatment beam and said magnetic field can be configured to rotate with respect to said target. This document discloses the use of an inline design approach.

Despite the good results already obtained by apparatuses from prior art, there is still a need for improvement of the performances of particle therapy apparatus comprising such a real time imaging system. For example, there is a need for further narrowing the particle beam in order to have a more precise delivery of high dose of energy in the target volume. There is also a need for an efficient apparatus integrating a particle therapy apparatus and a MRI system that is more compact.

One of the objectives of the invention is to provide a compact solution to at least one of the drawbacks of prior art.

### Summary of the invention

According to a first aspect, the invention relates to a particle therapy apparatus for irradiating a target with a charged particle beam comprising:
- a particle accelerator to generate the charged particle beam;
- an isocentric gantry configured to rotate about an axis Y and comprising a plurality of bending magnets arranged along a beam path, said plurality of magnets comprising a first bending magnet and a last bending magnet, the first bending magnet being configured to receive the particle beam along the axis Y and to bend and direct the particle beam away from the axis Y, the last bending magnet being configured to bend and direct the particle beam towards the isocentre of the gantry and according to a final beam direction;
- a magnetic resonance imaging system comprising a first and a second main magnet unit separated by a free air gap and arranged respectively on opposites sides of the gantry's isocentre, the first main magnet unit being the one closest to the last bending magnet of the gantry, said first and second main magnet units being configured to generate together a main magnetic field (Bo) which is parallel to the final beam direction at the gantry's isocentre,
wherein the apparatus further comprises a passive magnetic shield surrounding the first and second main magnet units of the magnetic resonance imaging system so that a part of the passive magnetic shield is arranged between the last bending magnet of the gantry and the first main magnet unit of the magnetic resonance imaging system, wherein the said part of the passive magnetic shield comprising a first through hole defining a passageway through which the particle beam can pass to reach the gantry's isocentre, and further wherein the passive magnetic shield as well as the first and the second main magnet units of the magnetic resonance imaging system are all synchronously rotatable with the gantry about the axis Y.

With preference, one or more of the following features can be used to further define the inventive apparatus or its embodiments:
- The final beam direction is a direction perpendicular to the axis Y;
- The first and second main magnet units of the MRI system and/or the passive magnetic shield are/is fixed to the gantry so as to rotate synchronously with the gantry when the gantry is put into rotation;
- The apparatus further comprises at least one scanning magnet arranged on the gantry along the beam path - preferably between the first and the last bending magnet of the gantry - and configured to scan the particle beam over the target;
- The particle beam is a beam of charged particles chosen among protons or ions.
- The particle accelerator is a cyclotron or a synchrotron;
- Each of the first and second main magnet units of the magnetic resonance imaging system comprises a superconducting electromagnet;
- The isocentre of the gantry coincides with the imaging centre of the magnetic resonance imaging system.

With preference, one or more of the following features can be used to further define the passive magnetic shield according to the invention:
- The passive magnetic shield is designed to be unsaturated by the magnetic fields of the magnetic resonance imaging system and/or by the magnetic fields of the magnets of the gantry (bending magnets and/or scanning magnets);
- The passive magnetic shield is designed to be unsaturated by the magnetic fields of the magnetic resonance imaging system and/or by the fields of both the scanning magnets and the bending magnets of the gantry;
- The passive magnetic shield comprises a second through hole facing the first through hole along the final beam direction;
- The passive magnetic shield comprises a third through hole whose axis is parallel to or coincides with the axis Y and which is dimensioned to allow a passage of a patient through it;
- The passive magnetic shield comprises a fourth through hole facing the third through hole along the Y axis;
- The passive magnetic shield comprises at least one yoke of ferromagnetic material;
- The said yoke has a parallelepiped shape;
- The first through hole has a diameter comprised in the range of 10 cm to 50 cm.

The apparatus according to the invention provide a more compact solution to the prior art apparatus.

### Description of the figures

Figure 1 is a view of a part of an apparatus of the invention according to a first embodiment;
Figure 2 is a cross sectional view of a part of the apparatus of figure 1;
Figure 3 is a perspective view of a part of an apparatus according to a preferred embodiment of the invention.

The figures are neither drawn to scale nor proportioned. Generally, similar or identical components are denoted by the same reference numerals in the figures.

### Detailed description of the invention

For the purpose of the invention, the terms "comprising", "comprises" and "comprised of" as used herein are synonymous with "including", "includes" or "containing", "contains", and are inclusive or open-ended and do not exclude additional, non-recited members, elements or method steps. The terms "comprising", "comprises" and "comprised of" also include the term "consisting of".

The recitation of numerical ranges by endpoints includes all numbers and fractions subsumed within the respective ranges, as well as the recited endpoints.

The particular features, structures, characteristics or embodiments may be combined in any suitable manner, as would be apparent to a person skilled in the art from this disclosure, in one or more embodiments.

A particle therapy apparatus for irradiating a target with a charged particle beam for therapy purposes is generally widely known from the prior art and comprises a particle accelerator to generate the charged particle beam, a beam transport system to transport the particle beam from the particle accelerator to the target to be irradiated, and various other subsystems, for example to shape the beam and/or to modify its energy and/or its intensity for the particular therapy envisaged. The target may for example be a diseased part such as a tumor in a patient's body. Hence, the term "target" used hereinafter may refer as well to such a tumor as to the patient himself.

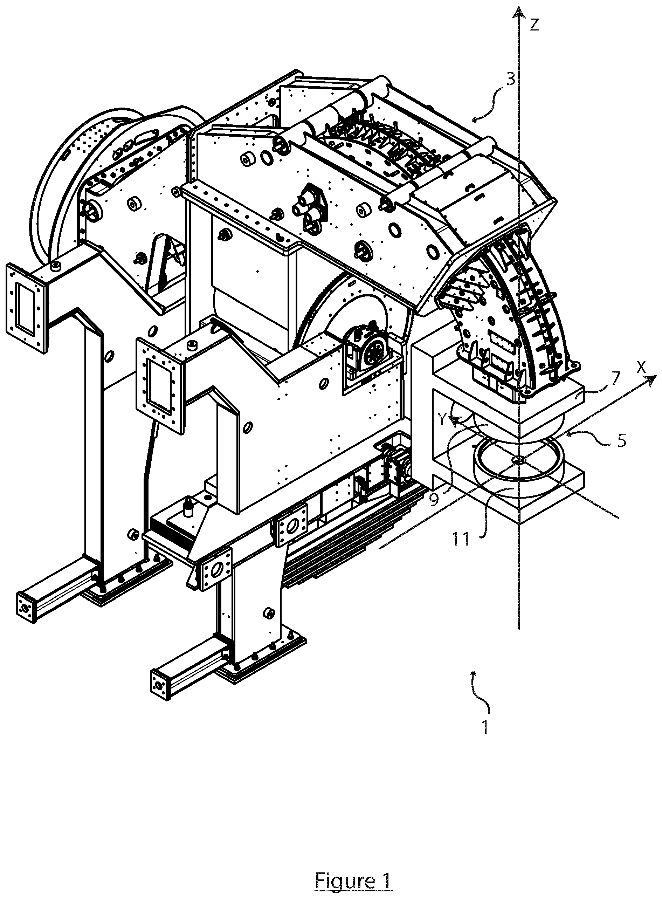

Reference is now made to figure 1, which shows a part of a particle therapy apparatus 1 according to an exemplary embodiment of the invention. It comprises a particle accelerator (not shown for the sake of clarity) to generate the charged particle beam, an isocentric gantry 3 which is rotatable about an axis Y in the shown orthogonal XYZ referential, and a part of a beam transport system (also not shown for the sake of clarity) to transport the particle beam, from where it is extracted from the particle accelerator to an entry point of the particle beam into a gantry beam line. In the present case, the particle beam enters the gantry beam line in parallel to or coincident with the rotation axis Y of the gantry. The rotatable gantry 3 is said to be isocentric because the particle beams exiting from the last bending magnet for any angle of rotation of the gantry will all cross at a same point called the "isocentre", hereinafter sometimes referred to as the isocentre of the gantry. In practice, and as is well known, due to the heavy weight and mechanical imperfections of the system, the isocentre is strictly speaking not a single point but rather a small sphere. On figure 1, the isocentre is the origin of the XYZ referential.

Preferably, the gantry 3 is rotatable over an angular range of at least 180° and more preferably over an angular range up to 360°.

The rotatable gantry 3 comprises and supports a plurality of bending magnets arranged in sequence along a beam path and including a first bending magnet and a last bending magnet. The first bending magnet of said sequence is configured to receive the particle beam along the axis Y and to bend and direct the particle beam away from the axis Y. The last bending magnet of said sequence is configured to bend and direct the particle beam towards the isocentre. The said sequence may comprise one or more additional bending magnets arranged on the gantry along the beam path between the first bending magnet and the last bending magnet.

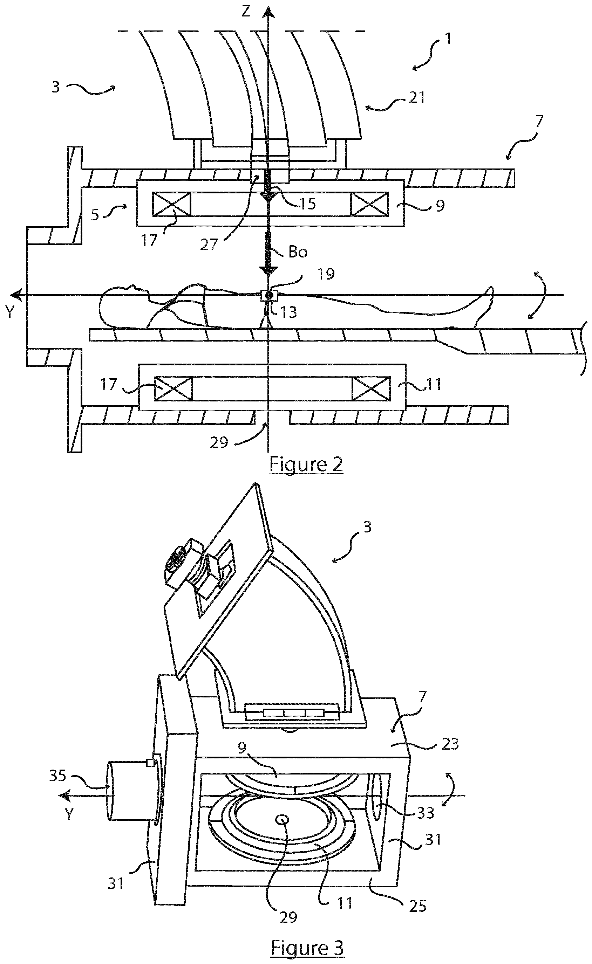

According to the invention, the apparatus further comprises a magnetic resonance imaging system 5 including a first main magnet unit 9 and a second main magnet unit 11. The first and second main magnet units are separated by a free air gap and are arranged respectively on opposite sides of the gantry's isocentre. Preferably, the first and second main magnet units are arranged symmetrically with respect to the gantry's isocentre. The first main magnet unit 9 is the one located closest to the last bending magnet of the gantry 3. The first 9 and second 11 main magnet units are arranged and configured to generate together a main magnetic field (Bo) which is parallel to the final beam direction at the gantry's isocentre, as will appear more clearly on figure 2.

According to the invention, the apparatus further comprises a passive magnetic shield 7 surrounding the first 9 and second 11 main magnet units of the magnetic resonance imaging system.

Reference is now made to figure 2, which is a partial cross-sectional view of the apparatus of figure 1. Figure 2 shows a part of the last bending magnet 21 of the gantry 3 of the apparatus 1. As one can see on this figure, the last bending magnet 21 is configured to bend and direct the particle beam 15 towards the isocentre 19 of the gantry and according to a final beam direction. In this example, the final beam direction is a direction perpendicular to the axis Y, whatever the rotation angle of the gantry. In another embodiment (not illustrated) the last bending magnet of the gantry is configured to bend and direct the particle beam 15 towards the isocentre 19 of the gantry and according to a final beam direction which is making an angle alpha with the axis Y such that alpha is larger than 70 degrees and smaller than 90 degrees, preferably larger than 80 degrees and smaller than 90 degrees, whatever the rotation angle of the gantry.

The MRI system 5 comprises a first 9 and a second 11 main magnet units which are separated by an air gap and which are arranged respectively on opposite sides of the gantry's isocentre 19. Each of the first 9 and second 11 main magnet units comprises a magnet 17, preferably an electromagnet, more preferably a superconducting electromagnet, which, when excited, generate together the main magnetic field Bo. As shown on figure 2, the first 9 and second 11 main magnet units are arranged and configured to generate together a main magnetic field Bo which is parallel to the final beam direction at the gantry's isocentre and in the close vicinity thereof. As is known from the art, such main magnet units have the general shape of an open torus. As shown on figure 2, the two tori are arranged in such a way that their axis of revolution are both parallel to the final beam direction. Preferably, their axis of revolution are identical. In any case, the first 9 and a second 11 main magnet units are arranged in such a way that they are not in the way of the path of the particle beam.

Preferably, the first 9 and a second 11 main magnets of the MRI system are further arranged in such a way that the imaging center of the MRI system coincides with isocentre 19 of the gantry 3. MRI images of the target 13 can then be taken at the isocentre. Such images may for example be used to track the position of the target in real time before, while, or after irradiating the target with the particle beam, and possibly to correct the beam path and/or the beam energy and/or the beam intensity in function thereof.

In a preferred embodiment, the particle therapy apparatus according to the invention further comprises at least one scanning magnet (not represented) arranged along the beam path and configured to scan the particle beam over the target. Such scanning magnets are known in the art, for example in the art of the pencil beam scanning technique (PBS), and will not be described here any further. Preferably, at least one such scanning magnet is arranged on the gantry between the first and the last bending magnet 21.

In accordance with the invention, the apparatus 1 further comprises a passive magnetic shield 7 surrounding the first 9 and second 11 main magnet units of the magnetic resonance imaging system 5, the first main magnet unit 9 being the one closest to the last bending magnet 21 of the gantry 3, as shown on figure 2. At least a part of the passive magnetic shield is arranged between the last bending magnet 21 of the gantry 3 and the first main magnet unit 9 of the MRI system 5. Said part of the passive magnetic shield comprises a first through hole 27 defining a passageway through which the particle beam 15 can pass to reach the gantry's isocentre 19. Preferably, said first through hole 27 has a diameter ranging from 10 cm to 50 cm, more preferably ranging from 20 cm to 40 cm.

In a preferred embodiment of the invention, the passive magnetic shield 7 comprises at least one yoke of ferromagnetic material, such as iron, nickel, cobalt and their alloys (for example the mu-metal). In an embodiment, the passive magnetic shield 7 comprises one yoke. In another embodiment, the passive magnetic shield 7 comprises two or more yokes being concentric and nested ones within the others.

Preferably, the yoke 7 has a parallelepiped shape, more preferably a rectangular parallelepiped shape. In the embodiment illustrated on figure 1 and 2, the yoke 7 has a rectangular parallelepiped shape, and presents three faces: a superior base comprising the first through hole 27 and an inferior base which are both parallel to the Y axis, and one lateral face which is perpendicular to the Y axis and is located at the left side of the isocentre (at the side of the gantry entry point). Such a "laying U" configuration is considered to be a minimal one in order to have an efficient passive magnetic shielding in accordance to the invention.

In a preferred embodiment, as illustrated on figure 3, the yoke has four faces: a superior base 23 and an inferior base 25, which are both parallel to the Y axis, and two lateral faces 31, which are both perpendicular to said Y axis. The superior base 23 is located between the last bending magnet 21 of the gantry 3 and the first main magnet 9 of the MRI system. Said superior base 23 comprises the first through hole through which the particle beam can pass to reach the gantry's isocentre.

Thus, referring to figures 2 and 3, the yoke 7 has preferably an open configuration, wherein one, two or three of its lateral faces are open. It has been found by the inventors that such laterally opened configuration is sufficient to allow the yoke to be used as passive magnetic shield, yet allowing the yoke to be lighter in weight and more comfortable and less stressful for patients suffering for example from claustrophobia.

In a preferred embodiment, the passive magnetic shield 7 further comprises a second through hole 29 facing the first through hole 27 along the final beam direction Preferably the second through hole 29 has same diameter as the first through hole 27. In the embodiments of figures 2 and 3, the second through hole is a hole in the inferior base of the magnetic shield. This second through hole allows to further image the target from a point of view located under the said inferior base.

In the embodiment of figure 3, the passive magnetic shield 7 preferably further comprises a third through hole 33 whose axis coincides with the axis Y and which is dimensioned to allow a passage of a patient through it. More preferably, the passive magnetic shield 7 comprises a fourth through hole 35 facing the third through hole 33 along the Y axis. In the embodiment of figure 3, the third 33 and the fourth through holes 35 are located respectively on the two lateral faces 31 of the yoke and are coaxial with the Y axis.

Preferably, at least one lateral face of the yoke is higher in size that the external distance between the two bases.

Whatever the embodiment, the passive magnetic shield 7 is designed and dimensioned to be unsaturated by the magnetic fields generated by the MRI system and/or to be unsaturated by the magnetic fields generated by the magnetic elements of the gantry, such as the bending magnets and/or the scanning magnets for example.

According to the invention, the passive magnetic shield 7 as well as the first 9 and second 11 main magnet units of the MRI system 5 are all synchronously rotatable about the axis Y with the gantry 3. In other words, the passive magnetic shield 7 as well as the first 9 and second 11 main magnet units of the MRI system 5 are all rotatable about the axis Y at the same rotational speed and with the same phase as the gantry 3. This can be achieved in several ways. One way is to make use of three motors : a first one for rotating the gantry, a second one for rotating the magnetic shield and a third one for rotating the first and second main magnet units of the MRI system. Evidently, the three motors must then be synchronized. Alternatively, two motors may be used : a first one for rotating the gantry and second one for rotating both the magnetic shield and the first and second main magnet units of the MRI system together, which can be realized by attaching together the magnetic shield and the first and second main magnet units of the MRI system. Evidently, the two motors must then be synchronized. Preferably, a single motor is used for rotating together the gantry 3, the magnetic shield 7, and the first 9 and second 11 main magnet units of the MRI system, which can be realized by attaching all these subsystems together and by operatively connecting one of them to the motor.

In a preferred embodiment, the first 9 and second 11 main magnet units are fixed to the passive magnetic shield 7 and the passive magnetic shield 7 is fixed to the gantry 3, so that there is no relative movement between the gantry 3, the passive magnetic shield 7 and the two main magnet units of the MRI system 5. In such a case, a single motor is provided to rotate these three subsystems together. Preferably, this single motor is operatively connected to the gantry.

Rollers may be provided in order to sustain the passive magnetic shield, which is generally quite heavy.

Preferably, the particle beam is a beam of electrically charged particles, excluding electrons. More preferably, the particle beam is a beam of protons or a beam of carbon ions. Preferably, the particle accelerator is a cyclotron or a synchrotron, more preferably a synchrocyclotron, even more preferably a superconducting synchrocyclotron. Preferably, the particle accelerator is adapted to generate and deliver a beam of charged particles whose energy is higher than 60 MeV.

The invention allows lowering the magnetic interaction and/or the perturbation between the MRI and particle treatment subsystems, so that the particle therapy apparatus shows improved performances and/or can be more compact.

## Claims

1. A particle therapy apparatus (1) for irradiating a target (13) with a charged particle beam (15) comprising:
- a particle accelerator to generate the charged particle beam (15);
- an isocentric gantry (3) configured to rotate about an axis Y and comprising a plurality of bending magnets arranged along a beam path, said plurality of magnets comprising a first bending magnet and a last bending magnet (21), the first bending magnet being configured to receive the particle beam (15) along the axis Y and to bend and direct the particle beam (15) away from the axis Y, the last bending magnet (21) being configured to bend and direct the particle beam (15) towards the isocentre of the gantry (3) and according to a final beam direction;
- a magnetic resonance imaging system (5) comprising a first (9) and a second (11) main magnet unit separated by a free air gap and arranged respectively on opposites sides of the gantry's isocentre (19), the first main magnet unit (9) being the one closest to the last bending magnet (21) of the gantry (3), said first (9) and second (11) main magnet units being configured to generate together a main magnetic field (Bo) which is parallel to the final beam direction at the gantry's isocentre,
**characterized in that** the apparatus (1) further comprises a passive magnetic shield (7) surrounding the first (9) and second (11) main magnet units of the magnetic resonance imaging system (5) and so that a part of the passive magnetic shield (7) is arranged between the last bending magnet (21) of the gantry (3) and the first main magnet unit (9) of the magnetic resonance imaging system (5), wherein the said part of the passive magnetic shield (7) comprises a first through hole (27) defining a passageway through which the particle beam (15) can pass to reach the gantry's isocentre, and **in that** the passive magnetic shield (7) as well as the first (9) and second (11) main magnet units of the magnetic resonance imaging system (5) are all synchronously rotatable with the gantry (3) about the axis Y.

2. The particle therapy apparatus according to claim 1, **characterized in that** the final beam direction is a direction perpendicular to the axis Y.

3. The particle therapy apparatus (1) according to any one of claims 1 or 2 **characterized in that** the first (9) and second (11) main magnet units are fixed to the passive magnetic shield, and/or **in that** the passive magnetic shield (7) is fixed to the gantry (3).

4. The particle therapy apparatus (1) according to any one of claims 1 to 3, **characterized in that** the passive magnetic shield (7) is designed to be unsaturated by the magnetic fields of the magnetic resonance imaging system (5) and/or by the magnetic fields of magnets of the gantry (3).

5. The particle therapy apparatus (1) according to any one of claims 1 to 4, **characterized in that** the apparatus (1) further comprises at least one scanning magnet arranged along the beam path and configured to scan the particle beam over the target (13).

6. The particle therapy apparatus (1) according to claim 5, **characterized in that** the at least one scanning magnet is arranged on the gantry (3) between the first bending magnet and the last (21) bending magnet.

7. The particle therapy apparatus (1) according to any of previous claims, **characterized in that** the passive magnetic shield (7) comprises a second through hole (29) facing the first through hole (27) along the final beam direction.

8. The particle therapy apparatus (1) according to any of previous claims, **characterized in that** the passive magnetic shield (7) comprises a third through hole (33) whose axis is parallel to or coincides with the axis Y and which is dimensioned to allow a passage of a patient through it.

9. The particle therapy apparatus (1) according to any one of previous claims, **characterized in that** the passive magnetic shield (7) comprises at least one yoke of ferromagnetic material, with preference the yoke has a parallelepiped shape.

10. The particle therapy apparatus (1) according to any one of previous claims, **characterized in that** the first through hole (27) has a diameter comprised in the range of 10 cm to 50 cm.

11. The particle therapy apparatus (1) according to any one of previous claims **characterized in that** the particle beam (15) is a beam of charged particles chosen among protons or ions.

12. The particle therapy apparatus (1) according to any one of previous claims **characterized in that** the particle accelerator is a cyclotron or a synchrotron.

13. The particle therapy apparatus (1) according to any one of previous claims **characterized in that** each of the first (9) and second (11) main magnet units of the magnetic resonance imaging system (5) comprises a superconducting electromagnet.

14. The particle therapy apparatus (1) according to any one of previous claims **characterized in that** the isocentre of the gantry (3) coincides with an imaging center (19) of the magnetic resonance imaging system (5).

## Patentansprüche

1. Teilchentherapiegerät (1) zum Bestrahlen eines Ziels (13) mit einem geladenen Teilchenstrahl (15), umfassend:
- einen Teilchenbeschleuniger zum Generieren des geladenen Teilchenstrahls (15);
- ein isozentrisches Gerüst (3), das konfiguriert ist, um um eine Y-Achse zu rotieren und eine Vielzahl von Biegemagneten umfasst, die entlang einem Strahlenpfad angeordnet sind, wobei die genannte Vielzahl von Magneten einen ersten Biegemagneten und einen letzten Biegemagneten (21) umfasst, wobei der erste Biegemagnet konfiguriert ist, um den Teilchenstrahl (15) entlang der Y-Achse zu empfangen und den Teilchenstrahl (15) von der Y-Achse weg zu biegen und zu richten, wobei der letzte Biegemagnet (21) konfiguriert ist, um den Teilchenstrahl (15) zu dem Isozentrum des Gerüsts (3) und gemäß einer finalen Strahlenrichtung zu biegen und zu richten;
- ein Magnetresonanzsystem (5), umfassend eine erste (9) und eine zweite (11) Hauptmagneteinheit, die durch eine freie Luftlücke getrennt und jeweils auf entgegengesetzten Seiten des Isozentrums (19) des Gerüst angeordnet sind, wobei die erste Magneteinheit (9) diejenige ist, die dem letzten Biegemagnet (21) des Gerüsts (3) am nächsten ist, wobei die erste (9) und zweite (11) Magneteinheit konfiguriert sind, um zusammen ein Hauptmagnetfeld (Bo) zu generieren, das parallel zu der finalen Strahlenrichtung an dem Isozentrum des Gerüsts ist,
**dadurch gekennzeichnet, dass** das Gerät (1) weiterhin einen passiven Magnetschild (7) umfasst, der die genannte erste (9) und zweite (11) Magneteinheit des Magnetresonanz-Bildgebungssystems (5) umgibt, und derart, dass ein Teil des passiven Magnetschildes (7) zwischen dem letzten Biegemagnet (21) des Gerüsts (3) und der ersten Magneteinheit (9) des Magnetresonanz-Bildgebungssystems (5) angeordnet ist, wobei der genannte Teil des passiven Magnetschildes (7) ein erstes Durchgangsloch (27) umfasst, das einen Durchgang definiert, durch den der Teilchenstrahl (15) durchtreten kann, um das Isozentrum des Gerüsts zu erreichen, und dass der passive Magnetschild (7) sowie die erste (9) und zweite (11) Hauptmagneteinheit des Magnetresonanz-Bildgebungssystems (5) alle mit dem Gerüst (3) um die Y-Achse synchron rotierbar sind.

2. Teilchentherapiegerät gemäß Anspruch 1, **dadurch gekennzeichnet, dass** die finale Strahlenrichtung eine zur Y-Achse lotrechte Richtung ist.

3. Teilchentherapiegerät (1) gemäß irgendeinem der Ansprüche 1 oder 2, **dadurch gekennzeichnet, dass** die erste (9) und zweite (11) Hauptmagneteinheit an dem passiven Magnetschild befestigt sind und / oder dass der passive Magnetschild (7) an dem Gerüst (3) befestigt ist.

4. Teilchentherapiegerät (1) gemäß irgendeinem der Ansprüche 1 bis 3, **dadurch gekennzeichnet, dass** der passive Magnetschild (7) dazu ausgelegt ist, durch die Magnetfelder des Magnetresonanz-Bildgebungssystems (5) und / oder durch die Magnetfelder von Magneten des Gerüsts (3) ungesättigt zu sein.

5. Teilchentherapiegerät (1) gemäß irgendeinem der Ansprüche 1 bis 4, **dadurch gekennzeichnet, dass** das Gerät (1) weiterhin wenigstens einen Scannermagneten umfasst, der entlang dem Strahlenpfad angeordnet und konfiguriert ist, um den Teilchenstrahl über dem Ziel (13) zu scannen.

6. Teilchentherapiegerät (1) gemäß Anspruch 5, **dadurch gekennzeichnet, dass** der wenigstens eine Scannermagnet auf dem Gerüst (3) zwischen dem ersten Biegemagnet und dem letzten (21) Biegemagnet angeordnet ist.

7. Teilchentherapiegerät (1) gemäß irgendeinem der voranstehenden Ansprüche, **dadurch gekennzeichnet, dass** der passive Magnetschild (7) ein zweites Durchgangsloch (29) gegenüber dem ersten Durchgangsloch (27) entlang der finalen Strahlenrichtung umfasst.

8. Teilchentherapiegerät (1) gemäß irgendeinem der voranstehenden Ansprüche, **dadurch gekennzeichnet, dass** der passive Magnetschild (7) ein drittes Durchgangsloch (33) umfasst, dessen Achse parallel zu der Y-Achse ist oder damit zusammenfällt und die dazu ausgelegt ist, einen Durchgang eines Patienten dadurch zu erlauben.

9. Teilchentherapiegerät (1) gemäß irgendeinem der voranstehenden Ansprüche, **dadurch gekennzeichnet, dass** der passive Magnetschild (7) wenigstens ein Joch aus ferromagnetischem Material umfasst, wobei das Joch bevorzugt eine parallelflache Form hat.

10. Teilchentherapiegerät (1) gemäß irgendeinem der voranstehenden Ansprüche, **dadurch gekennzeichnet, dass** das erste Durchgangsloch (27) einen im Bereich von 10 cm bis 50 cm inbegriffenen Durchmesser hat.

11. Teilchentherapiegerät (1) gemäß irgendeinem der voranstehenden Ansprüche, **dadurch gekennzeichnet, dass** der Teilchenstrahl (15) ein Strahl aus geladenen Teilchen ist, die aus Protonen oder Ionen ausgewählt sind.

12. Teilchentherapiegerät (1) gemäß irgendeinem der voranstehenden Ansprüche, **dadurch gekennzeichnet, dass** der Teilchenbeschleuniger ein Zyklotron oder ein Synchroton ist.

13. Teilchentherapiegerät (1) gemäß irgendeinem der voranstehenden Ansprüche, **dadurch gekennzeichnet, dass** jede der ersten (9) und zweiten (11) Hauptmagneteinheiten des Magnetresonanz-Bildgebungssystems (5) einen supraleitenden Elektromagnet umfasst.

14. Teilchentherapiegerät (1) gemäß irgendeinem der voranstehenden Ansprüche, **dadurch gekennzeichnet, dass** das Isozentrum des Gerüsts (3) mit einem Bildgebungszentrum (19) des Magnetresonanz-Bildgebungssystems (5) zusammenfällt.

## Revendications

1. Appareil de thérapie par particules (1) pour irradier une cible (13) avec un faisceau de particules chargées (15), comprenant:
- un accélérateur de particules pour générer le faisceau de particules chargées (15);
- un portique isocentrique (3) configuré de manière à tourner autour d'un axe Y et comprenant une pluralité d'aimants de courbure agencés le long d'un chemin de faisceau, ladite pluralité d'aimants comprenant un premier aimant de courbure et un dernier aimant de courbure (21), le premier aimant de courbure étant configuré de manière à recevoir le faisceau de particules (15) le long de l'axe Y et à courber et à diriger le faisceau de particules (15) à l'écart de l'axe Y, le dernier aimant de courbure (21) étant configuré de manière à courber et à diriger le faisceau de particules (15) en direction de l'isocentre du portique (3) et suivant une direction de faisceau finale;
- un système d'imagerie par résonance magnétique (5) comprenant une première (9) et une seconde (11) unités d'aimant principales séparées par une couche d'air libre et agencés respectivement sur des côtés opposés de l'isocentre (19) du portique, la première unité d'aimant principale (9) étant l'unité la plus proche du dernier aimant de courbure (21) du portique (3), lesdites première (9) et seconde (11) unités d'aimant principales étant configurées de manière à générer ensemble un champ magnétique principal (Bo) qui est parallèle à la direction de faisceau finale au niveau de l'isocentre du portique,
**caractérisé en ce que** l'appareil (1) comprend en outre une bouclier magnétique passif (7) qui entoure les première (9) et seconde (11) unités d'aimant principales du système d'imagerie par résonance magnétique (5) et de telle sorte qu'une partie du bouclier magnétique passif (7) soit agencée entre le dernier aimant de courbure (21) du portique (3) et la première unité d'aimant principale (9) du système d'imagerie par résonance magnétique (5), dans lequel ladite partie du bouclier magnétique passif (7) comporte un premier trou traversant (27) qui définit un passage à travers lequel le faisceau de particules (15) peut passer pour atteindre l'isocentre du portique, et **en ce que** le bouclier magnétique passif (7) ainsi que les première (9) et seconde (11) unités d'aimant principales du système d'imagerie par résonance magnétique (5) sont toutes rotatives de façon synchronisée avec le portique (3) autour de l'axe Y.

2. Appareil de thérapie par particules selon la revendication 1, **caractérisé en ce que** la direction de faisceau finale est une direction perpendiculaire à l'axe Y.

3. Appareil de thérapie par particules (1) selon l'une quelconque des revendications 1 ou 2 **caractérisé en ce que** les première (9) et seconde (11) unités d'aimant principales sont fixées au bouclier magnétique passif, et/ou **en ce que** le bouclier magnétique passif (7) est fixé au portique (3).

4. Appareil de thérapie par particules (1) selon l'une quelconque des revendications 1 à 3, **caractérisé en ce que** le bouclier magnétique passif (7) est conçu de manière à être insaturé par les champs magnétiques du système d'imagerie par résonance magnétique (5) et/ou par les champs magnétiques des aimants du portique (3).

5. Appareil de thérapie par particules (1) selon l'une quelconque des revendications 1 à 4, **caractérisé en ce que** l'appareil (1) comprend en outre au moins un aimant de balayage agencé le long du chemin de faisceau et configuré de manière à balayer le faisceau de particules sur la cible (13).

6. Appareil de thérapie par particules (1) selon la revendication 5, **caractérisé en ce que** ledit au moins un aimant de balayage est agencé sur le portique (3) entre le premier aimant de courbure et le dernier (21) aimant de courbure.

7. Appareil de thérapie par particules (1) selon l'une quelconque des revendications précédentes, **caractérisé en ce que** le bouclier magnétique passif (7) comporte un deuxième trou traversant (29) en face du premier trou traversant (27) le long de la direction de faisceau finale.

8. Appareil de thérapie par particules (1) selon l'une quelconque des revendications précédentes, **caractérisé en ce que** le bouclier magnétique passif (7) comporte un troisième trou traversant (33) dont l'axe est parallèle à ou coïncide avec l'axe Y et qui est dimensionné de manière à permettre le passage d'un patient à travers celui-ci.

9. Appareil de thérapie par particules (1) selon l'une quelconque des revendications précédentes, **caractérisé en ce que** le bouclier magnétique passif (7) comprend au moins une armature de matériau ferromagnétique, l'armature présentant de préférence une forme parallélépipédique.

10. Appareil de thérapie par particules (1) selon l'une quelconque des revendications précédentes, **caractérisé en ce que** le premier trou traversant (27) présente un diamètre compris dans la gamme de 10 cm à 50 cm.

11. Appareil de thérapie par particules (1) selon l'une quelconque des revendications précédentes, **caractérisé en ce que** le faisceau de particules (15) est un faisceau de particules chargées choisies parmi des protons ou des ions.

12. Appareil de thérapie par particules (1) selon l'une quelconque des revendications précédentes, **caractérisé en ce que** l'accélérateur de particules est un cyclotron ou un synchrotron.

13. Appareil de thérapie par particules (1) selon l'une quelconque des revendications précédentes **caractérisé en ce que** chacune des première (9) et seconde (11) unités d'aimant principales du système d'imagerie par résonance magnétique (5) comprend un électroaimant supraconducteur.

14. Appareil de thérapie par particules (1) selon l'une quelconque des revendications précédentes, **caractérisé en ce que** l'isocentre du portique (3) coïncide avec un centre d'imagerie (19) du système d'imagerie par résonance magnétique (5).
